Europäisches Patentamt

European Patent Office

Office européen des brevets

(19)

(11) Numéro de publication : **0 346 181 B1**

(12)

# FASCICULE DE BREVET EUROPEEN

(45) Date de publication du fascicule du brevet :
**21.08.91 Bulletin 91/34**

(51) Int. Cl.⁵ : **G06F 15/62**

(21) Numéro de dépôt : **89401458.8**

(22) Date de dépôt : **29.05.89**

(54) **Procédé et système d'étalonnage d'un scanner à rayons x en utilisant un ou plusieurs étalons circulaires excentrés.**

(30) Priorité : **10.06.88 FR 8807791**

(43) Date de publication de la demande :
**13.12.89 Bulletin 89/50**

(45) Mention de la délivrance du brevet :
**21.08.91 Bulletin 91/34**

(84) Etats contractants désignés :
**DE ES GB IT NL**

(56) Documents cités :
**US-A- 4 352 020**
**US-A- 4 654 796**

(73) Titulaire : **GENERAL ELECTRIC CGR S.A.**
**100, rue Camille-Desmoulins**
**F-92130 Issy les Moulineaux (FR)**

(72) Inventeur : **Cornuejols, Dominique Cabinet**
**Ballot-Schmit**
**84, Avenue Kléber**
**F-75116 Paris (FR)**
Inventeur : **Feldman, Andrei Cabinet**
**Ballot-Schmit**
**84, Avenue Kléber**
**F-75116 Paris (FR)**

(74) Mandataire : **Ballot, Paul Denis Jacques**
**Cabinet Ballot-Schmit 7, rue le Sueur**
**F-75116 Paris (FR)**

## Description

L'invention concerne les scanners à rayons X et, plus particulièrement un procédé d'étalonnage d'appareils de ce type qui utilise un ou plusieurs étalons circulaires excentrés par rapport à l'axe de rotation du scanner ; elle concerne également un système qui permet de mettre en oeuvre ce procédé.

Pour examiner un patient, on utilise de plus en plus des appareils à rayons X appelés "scanners" qui réalisent des images de coupes transversales du patient. Ces appareils sont basés sur le phénomène physique d'absorption des rayons X par le corps humain. Pour un faisceau monochromatique, cette absorption est directement liée à la distance parcourue x des rayons X dans un corps homogène selon la formule :

$$I = I_o e - bx$$

formule dans laquelle :
$I_o$ est l'intensité du rayonnement entrant dans le corps humain,
$I$ est l'intensité du rayonnement sortant du corps humain,
$b$ est un coefficient d'atténuation qui dépend du corps traversé.

Dans une échelle de mesure logarithmique, l'atténuation I/Io est égale à bx, c'est-à-dire qu'elle est proportionnelle à la distance x.

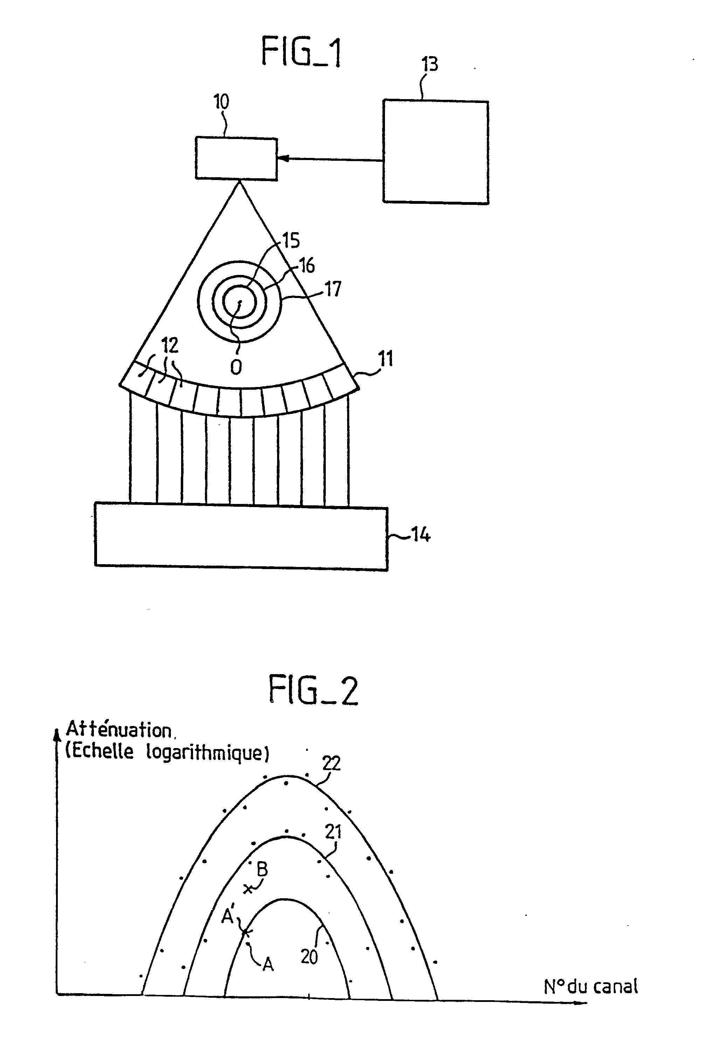

Ces appareils sont constitués essentiellement, comme le montre la figure 1, d'une source de rayons X référencée 10 qui est associée à un dispositif de détection 11, ces deux éléments étant disposés l'un par rapport à l'autre dans une relation géométrique fixe de manière à pouvoir intercaler entre eux le corps à examiner. En outre, ils sont supportés par une structure (non représentée) qui peut tourner autour du corps à examiner de manière à irradier le corps suivant des angles différents. La source de rayons X, qui est commandée par un dispositif 13, émet ses rayons suivant un secteur angulaire ayant une largeur suffisante pour illuminer toute la section transversale du corps. Le dispositif de détection 11 a la forme d'un secteur annulaire dont la longueur est adaptée à la largeur du faisceau de rayons X et est constitué d'un grand nombre de détecteurs élémentaires 12 juxtaposés les uns à côté des autres.

Pour obtenir une image de la section transversale du corps humain traversé par le faisceau de rayons X, on fait tourner la structure de support de la source 10 et du dispositif de détection 11 autour du corps et on mesure les signaux de sortie des détecteurs élémentaires 12 pour les traiter de manière appropriée selon des procédés connus afin d'en tirer une image représentative de la section transversale. Pour ce traitement, les détecteurs élémentaires 12, également appelés canaux, sont connectés à un dispositif électronique 14 qui effectue en premier lieu un calcul du logarithme des signaux reçus de manière à obtenir un signal dont l'amplitude est proportionnelle à l'atténuation des rayons X.

Par suite de l'effet de différents phénomènes qui ne seront pas expliqués ici, l'amplitude de ce signal pour chaque détecteur élémentaire ou canal n'est pas proportionnelle à l'atténuation effectivement subie. En conséquence, pour remédier à cet inconvénient, il a été imaginé divers procédés qui consistent par exemple à relever les signaux de sortie des canaux en présence de corps dont on connaît les dimensions et le coefficient d'absorption, de manière à calculer les atténuations (calculs de logarithmes) et à comparer ces atténuations mesurées à des valeurs calculées en fonction des dimensions et du coefficient d'absorption du corps ou étalon. Ces comparaisons permettent de déduire une loi de correspondance ou une loi modificatrice entre les valeurs mesurées et les valeurs que l'on devrait obtenir. Cette loi peut être sous la forme de fichiers de correspondance ou de formules mathématiques traduisant cette correspondance pour chaque canal de détection.

Les étalons qui sont utilisés pour effectuer ces mesures dites d'étalonnage sont par exemple des cales d'épaisseurs différentes qui sont introduites à proximité de la source de rayons X, ce qui implique des manipulations au niveau de la source pour introduire et retirer ces cales.

Dans le brevet US N° 4352020, il est proposé d'utiliser des cales ou étalons circulaires 15, 16 et 17, de différents diamètres, qui sont disposées au centre de rotation de la structure du support. Ceci permet de se rapprocher des conditions des mesures qui seront effectuées sur le corps à examiner. Ce brevet propose également d'utiliser un étalon en forme de cône à section circulaire qui est déplacé transversalement par rapport au faisceau de manière à obtenir différentes longueurs d'atténuation. Avec les étalons décrits, les mesures sont effectuées pour une position déterminée de la structure de support et par étalon.

La figure 2 montre l'allure de trois courbes de réponse 20, 21 et 22 de l'atténuation en fonction de la position des canaux dans le cas de mesures sur trois étalons de forme circulaire. Les valeurs mesurées sont représentées par les pointillés et varient autour d'une valeur moyenne qui représente la valeur théorique dans un système linéaire. Ces courbes peuvent être utilisées de la manière suivante ; lorsque le signal mesuré correspond à un point A, on en déduira que le signal linéaire est le point A' de la courbe moyenne 20. Lorsque le signal mesuré correspond à un point B situé entre les courbes 20 et 21, on en déduira le signal linéaire par interpolation entre les courbes 20 et 21. Cette interpolation peut être calculée suivant une loi linéaire ou plus généralement une loi polynomiale.

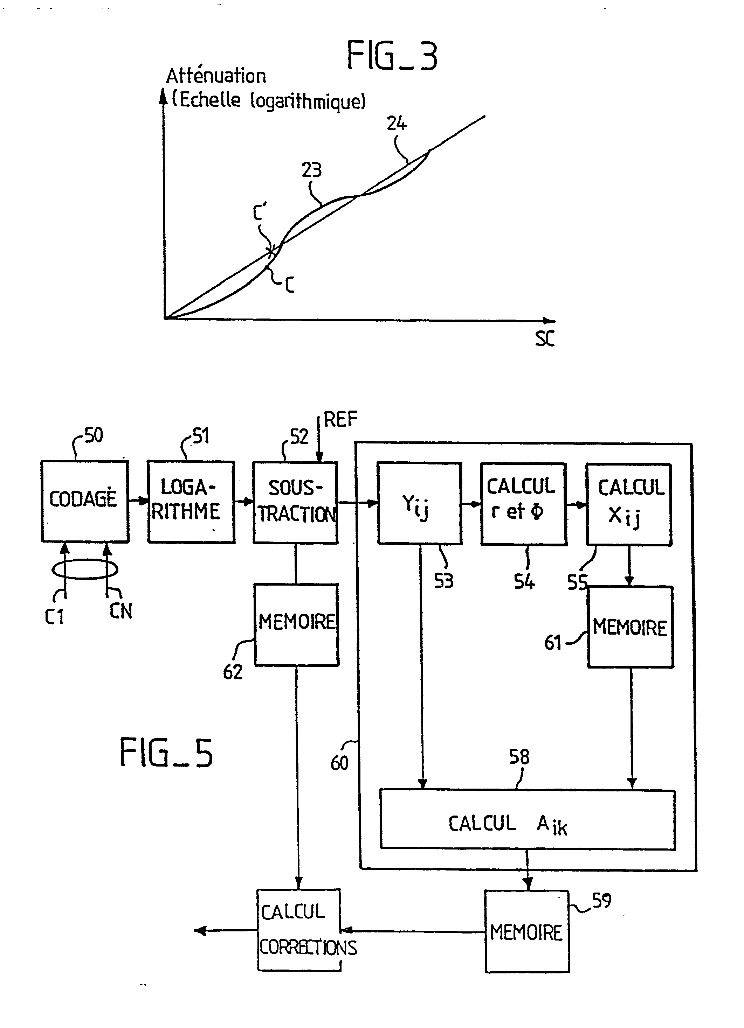

Les courbes 23 et 24 de la figure 3 montrent sous une autre forme le principe de l'étalonnage au niveau d'un canal. Il s'agit de courbes décrivant dans un canal donné l'atténuation en fonction de l'épaisseur x, pour des valeurs mesurées (courbe 23) et pour des valeurs calculées (droite 24) : en fait, les valeurs mesurées donnent des points, points que l'on relie entre eux selon une loi choisie : linéaire ou polynomiale, de manière à obtenir une courbe continue. Lorsqu'on mesure une atténuation, cela correspond par exemple au point C de la courbe 23 et on en déduit la valeur linéaire correspondant au point C′ de la courbe 24.

Le brevet américain précité décrit un appareil dans lequel la correspondance entre les valeurs mesurées et les valeurs réelles d'atténuation est effectuée par un système de fichiers créés pendant l'opération d'étalonnage. En ce qui concerne l'interpolation, le brevet propose des interpolations linéaires, cubiques et biquadratiques mais seule l'interpolation linéaire est décrite de manière détaillée.

Les procédés d'étalonnage qui ont été succinctement décrits ci-dessus présentent l'inconvénient majeur de mettre en oeuvre plusieurs étalons, ce qui conduit à de nombreuses manipulations ; en outre, ces manipulations doivent être précises, notamment dans le cas d'étalons circulaires dont les différents centres doivent coïncider avec le centre de rotation de la structure.

Il est à noter que le brevet américain propose d'utiliser un étalon unique qui aurait la forme donnée par la figure 12 dudit brevet, et de faire tourner la structure autour de cet étalon, ce qui conduit à obtenir des trajets d'absorption de longueur différente selon la position angulaire de la structure. Cependant, une telle manière de faire n'est que citée et n'indique pas le procédé ni les moyens de le mettre en oeuvre dans ce cas.

En résumé, le but des procédés d'étalonnage est d'homogénéiser la réponse des canaux entre eux et à tendre vers une réponse linéaire. Pour obtenir cette réponse linéaire, une correction polynomiale est appliquée à chaque canal et le calcul de cette correction, c'est-à-dire la détermination des coefficients du polynôme, s'effectue à partir de mesures effectuées avec des valeurs différentes du trajet d'absorption. C'est ainsi que pour déterminer les coefficients d'un polynôme d'ordre n d'un canal déterminé, il est nécessaire et suffisant d'effectuer (n + 1) mesures de trajets différents pour ce canal.

Dans les exemples de réalisation de l'art antérieur décrits ci-dessus, il a été montré que l'on utilise par exemple (n + 1) cales d'épaisseurs différentes ou (n + 1) cales circulaires de diamètres différents, les valeurs des épaisseurs et des diamètres étant choisis pour couvrir la dynamique requise. Il en résulte donc de nombreuses manipulations pour passer d'une cale à une autre, manipulations qui doivent être précises dans le cas de cales circulaires dont les centres doivent coïncider avec le centre de la structure.

Un but de la présente invention est donc de mettre en oeuvre un procédé d'étalonnage qui utilise un seul étalon circulaire dont la position est excentrée par rapport à l'axe de rotation du scanner.

Un autre but de la présente invention est de réaliser un appareil d'étalonnage pour mettre en oeuvre ledit procédé.

L'invention se rapporte à un procédé d'étalonnage d'un scanner à rayons X qui comporte une source de rayons X et un dispositif de détection de $i = 1$ à $N$ détecteurs ou canaux portés par une structure qui tourne autour d'un axe (O), en utilisant un étalon circulaire, caractérisé en ce qu'il comprend les opérations suivantes:

— la mise en place d'un étalon circulaire dont le centre (E) est placé à une position excentrée par rapport à l'axe de rotation du scanner,

— le déplacement du scanner de $j = 1$ à $m$ positions angulaires différentes $\beta_j$,

— le calcul, à partir des signaux reçus des détecteurs par canal de rang $i$ et pour chaque position angulaire $\beta_j$ du scanner, de signaux $Y_{ij}$ représentatifs de l'atténuation subie par le rayonnement X dans l'étalon,

— l'enregistrement des $Nxm$ signaux $Y_{ij}$,

— le calcul, à partir de signaux $r$ et $\Phi$ de décentrage, de signaux $X_{ij}$ représentatifs de la distance parcourue par le rayonnement X dans l'étalon, ainsi que de signaux d'atténuation correspondants $b \times X_{ij}$, $r$ et $\Phi$ étant les coordonnées polaires du centre (E) de l'étalon par rapport à un système de coordonnées lié audit axe de rotation (0), et $b$ étant le coefficient d'atténuation de l'étalon,

— la comparaison des signaux $Y_{ij}$ avec les signaux d'atténuation $b \times X_{ij}$ pour en déduire, pour chaque canal de rang $i$, des coefficients $A_{ik}$ du polynôme $P_n^i$ de degré $n$ tel que :

$$P_n^i (Y_{ij}) = \sum_{k=0}^{k=n} A_{ik} \; Y_{ij}^k$$

de manière à obtenir une approximation polynomiale des valeurs calculées $X_{ij}$ suivant une loi d'écart déterminée.

L'invention se rapporte également à un appareil d'étalonnage d'un scanner à rayons X qui comporte une source de rayons X et un dispositif de détection de i = 1 à N détecteurs ou canaux portés par une structure qui tourne autour d'un axe (0), en utilisant un étalon circulaire, caractérisé en ce qu'il comprend :

— des moyens pour disposer au moins un étalon circulaire entre la source de rayons X et le dispositif de détection dans une position excentrée par rapport à l'axe de rotation (0) de la structure tournante,

— des moyens pour déplacer le scanner de j = 1 à m positions angulaires différentes $\beta_j$,

— des moyens pour calculer pour chaque position $\beta_j$, à partir des signaux reçus sur les N détecteurs, N signaux $Y_{ij}$ représentatifs de l'atténuation subie par le rayonnement X dans l'étalon,

— des moyens pour enregistrer les N signaux $Y_{ij}$ pour chacune des m positions angulaires $\beta_j$,

— des moyens pour calculer pour toutes les positions angulaires j = 1 à m, à partir de signaux r et $\Phi$ de décentrage, des signaux $X_{ij}$ représentatifs de la longueur des trajets du rayonnement X dans l'étalon pour les canaux i = 1 à N ainsi que les signaux d'atténuation correspondants, b · $X_{ij}$, r et $\Phi$ étant les coordonnées polaires du centre (E) de l'étalon par rapport à un système de coordonnées lié audit axe de rotation (0), et b étant le coefficient d'atténuation de l'étalon,

— des moyens pour comparer les signaux $Y_{ij}$ avec les signaux d'atténuation b · $X_{ij}$ et pour calculer des signaux représentatifs de coefficients $A_{ik}$ du polynôme $P_n^i$ de degré n tel que :

$$P_n^i (Y_{ij}) = \sum_{k=o}^{k=n} A_{ik} Y_{ij}^k$$

de manière à obtenir une approximation polynomiale des valeurs calculées $X_{ij}$ suivant une loi d'écart déterminée,

— des moyens pour enregistrer lesdits coefficients $A_{ik}$ ainsi calculés de manière à les utiliser ensuite comme coefficients multiplicateurs des atténuations $Y_{ij}$ subies par le rayonnement X dans le corps du patient.

D'autres caractéristiques et avantages de la présente invention apparaîtront à la lecture de la description suivante d'un exemple particulier de réalisation, ladite description étant faite en relation avec les dessins joints dans lesquels :

— la figure 1 est un schéma de principe d'un scanner à rayons X qui montre un certain nombre d'étalons circulaires utilisés pour l'étalonnage, selon l'art antérieur,

— la figure 2 est un diagramme montrant différentes courbes d'atténuation en fonction de la position des détecteurs ou canaux et pour plusieurs valeurs du diamètre ;

— la figure 3 est un diagramme montrant les courbes d'atténuation théorique et mesurée pour un canal déterminé en fonction du trajet d'absorption,

— la figure 4 est un schéma essentiellement géométrique qui permet de comprendre le calcul des trajets du rayonnement X pour un détecteur déterminé lorsque, selon l'invention, on utilise un étalon circulaire excentré, et

— la figure 5 est un schéma fonctionnel d'un système de traitement des signaux de sortie des détecteurs mettant en oeuvre le procédé selon l'invention.

Les figures 1, 2 et 3 qui ont permis de définir l'art antérieur dans le préambule ne seront pas décrites à nouveau.

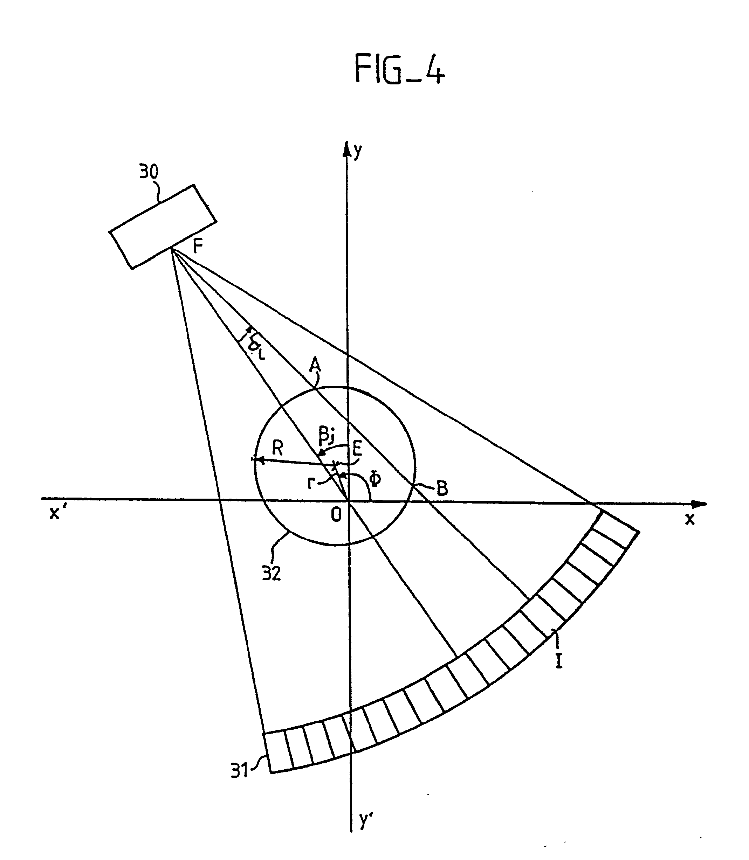

Sur la figure 4, on a représenté de manière schématique mais précise les positions d'une source de rayonnement X 30 matérialisé par le point F, d'un dispositif de détection 31 et d'un étalon circulaire 32 de rayon R disposé entre les deux premiers éléments 30 et 31. La source 30 et le dispositif de détection 31 sont portés par une structure, non représentée, qui tourne autour d'un axe perpendiculaire au plan du dessin au point O. Le centre E de l'étalon circulaire 32 est situé à une distance r du point O et la demi-droite OE fait un angle $\Phi$ avec l'axe Ox d'un système d'axes perpendiculaires x'x-y'y.

La position d'ordre j de la structure de support est définie par l'angle $\beta_j$ entre l'axe Oy et la demi-droite OF (position de la mesure ou de la vue) tandis que la position d'un détecteur I d'ordre i du dispositif de détection 31 est définie par l'angle $\delta_i$ entre FO et FI. Ce schéma géométrique permet de calculer le trajet AB appelé $X_{ij}$ d'un rayonnement X dans l'étalon circulaire excentré 32 selon la formule

$$X_{ij} = AB = 2 \sqrt{R^2 - \{|FO| \sin \delta_i - r \cos (\Phi - \beta_j - \delta_i)\}^2} \quad (1)$$

Le calcul des coefficients $A_{ik}$ du polynôme d'ordre n du canal ou détecteur i est effectué par minimisation du critère quadratique $C_i$ suivant tel que :

$$C_i = \sum_{j=1}^{m} \left\{ \sum_{k=o}^{n} (A_{ik} \; Y_{ij}) - b \cdot X_{ij} \right\}^2 \qquad (2)$$

formule dans laquelle :

m     est le nombre de vues

$Y_{ij}$     est le signal du canal de rang i pour la vue d'ordre j

$A_{ik}$     est le coefficient du polynôme $P_n^i$ du canal i tel que

$$P_n^i(Y_{ij}) = \sum_{k=o}^{k=n} A_{ik} \; Y_{ij}^{k}$$

$$P^i(Y) = A_{i,o} + A_{i,1} Y + \ldots A_{i,k} Y^k + \ldots A_{i,n} Y^n$$

Le procédé d'étalonnage selon l'invention comprend donc les opérations suivantes :

— la mise en place d'un étalon circulaire dont le centre E est excentré par rapport au centre O de rotation du dispositif source de rayonnement-détecteur, les coordonnées de E étant définies par r et $\Phi$ ;

— la réalisation d'un nombre m de mesures $Y_{ij}$ supérieur ou égal à n + 1 par canal de rang i si l'on souhaite effectuer une approximation polynomiale de rang n ;

— le calcul, pour chaque mesure $Y_{ij}$, de la distance $X_{ij}$ par application de la formule (1)

— le calcul des coefficients $A_{ik}$ du polynôme $P_n^i$ par minimisation du critère quadratique défini par la formule (2).

On remarquera que le nombre m est largement supérieur à n + 1 afin de tenir compte du bruit des mesures.

Pour mettre en oeuvre ce procédé d'étalonnage, l'invention propose un système qui sera maintenant décrit en relation avec la figure 5. Il comprend un convertisseur analogique/numérique 50 auquel sont appliqués les signaux de sortie C1 à CN des N détecteurs ou canaux du dispositif de détection. Les N codes numériques correspondant à une position ou vue de rang j sont appliqués à un circuit de calcul de logarithme 51 qui fournit, pour chaque canal i et chaque vue j, un code représentatif de l'atténuation subie dans l'étalon. Les N codes résultant de cette opération de calcul logarithmique sont appliqués à un circuit de soustraction 52 dans lequel on leur soustrait une valeur REF représentative de l'atténuation subie par le rayonnement X à l'extérieur de l'étalon, c'est-à-dire dans l'air. Cette valeur est obtenue à l'aide d'un détecteur appelé moniteur dont la position sur le dispositif de détection 11 est telle qu'il reçoit le rayonnement X sans atténuation par l'étalon.

Ce sont les codes qui résultent de cette soustraction qui constituent les mesures $Y_{ij}$ définies ci-dessus. Les N valeurs correspondant à une vue de rang j sont enregistrées dans deux mémoires, l'une référencée 53 pour le calcul des coefficients $A_{ik}$ conformément au procédé d'étalonnage et l'autre référencée 62, pour être corrigée en dehors des opérations d'étalonnage, à l'aide des coefficients $A_{ik}$.

Chaque vue correspondant à un angle $ß_j$ différent, N valeurs $Y_{ij}$ sont enregistrées dans les mémoires 53 et 62. La mémoire 53 est par exemple prévue pour enregistrer les N · m codes correspondant à m vues si l'on souhaite effectuer une approximation polynomiale d'ordre n (n < m). Un dispositif de calcul de cette approximation polynomiale comprend les éléments suivants regroupés dans le rectangle référencé 60. Un circuit de calcul 54 du décentrage de l'étalon, c'est-à-dire le calcul des coordonnées polaires r et $\Phi$ du centre E de l'étalon. Les résultats de ce calcul sont utilisés dans un circuit 55 pour calculer les distances $X_{ij}$ en appliquant la formule (1) pour les m positions angulaires $ß_j$ des n vues. On supposera que les calculs dans les circuits 54 et 55 sont effectués de manière numérique et, en conséquence, les distances $X_{ij}$ se présentent sous la forme de codes analogues aux codes des mesures $Y_{ij}$ et sont enregistrés dans une mémoire 61 analogue à la mémoire 53.

Les codes contenus dans les mémoires 53 et 61 sont utilisés dans un circuit 58 pour calculer les coefficients polynomiaux $A_{ik}$ pour chaque canal de rang i en appliquant la formule (2) afin d'obtenir une valeur minimale pour Ci. Ces coefficients sont enregistrés dans une mémoire 59 de manière à être utilisés dans un circuit 63 pour corriger les valeurs mesurées en présence du corps du patient.

Le calcul des coordonnées r et $\Phi$ du centre E de l'étalon peut être réalisé de différentes manières. L'une d'entre elles est basée sur le fait que l'atténuation la plus forte due à l'étalon correspond à un trajet diamétral, c'est-à-dire passant par le centre E. Ainsi, pour la vue d'ordre j correspondant à $ß_j = O$ (OF confondu avec l'axe Oy), on détermine le canal i dans lequel l'atténuation est maximale, ce qui conduit à connaître l'angle $\alpha'$ entre l'axe Oy et la demi-droite FE. De la même manière, on peut déterminer l'angle $\alpha''$ entre l'axe Ox' et la demi-droite FE pour la vue correspondant à $ß_j = 90°$ (ou 180°).

La connaissance de ces deux angles $\alpha'$ et $\alpha''$ associée à celle de la distance FO permet de calculer par les relations trigonométriques les coordonnées cartésiennes du centre E de l'étalon par rapport aux axes x'x et y'y. Une transformation de coordonnées cartésiennes en coordonnées polaires permet d'obtenir r et $\Phi$.

**Revendications**

1. Procédé d'étalonnage d'un scanner à rayons X qui comporte une source de rayons X (30) et un dispositif de détection (31) de i = 1 à N détecteurs ou canaux portés par une structure qui tourne autour d'un axe (O), en utilisant un étalon circulaire (32), caractérisé en ce qu'il comprend les opérations suivantes :
   — la mise en place d'un étalon circulaire (32) dont le centre (E) est placé à une position excentrée par rapport à l'axe de rotation du scanner,
   — le déplacement du scanner de j = 1 à m positions angulaires différentes $ß_j$,
   — le calcul, à partir des signaux reçus des détecteurs par canal de rang i et pour chaque position angulaire $ß_j$ du scanner, de signaux $Y_{ij}$ représentatifs de l'atténuation subie par le rayonnement X dans l'étalon,
   — l'enregistrement des Nxm signaux $Y_{ij}$,
   — le calcul, à partir de signaux r et $\Phi$ de décentrage, de signaux $X_{ij}$ représentatifs de la distance parcourue par le rayonnement X dans l'étalon, ainsi que de signaux d'atténuation correspondants $b \times X_{ij}$, r et $\Phi$ étant les coordonnées polaires du centre (E) de l'étalon par rapport à un système de coordonnées lié audit axe de rotation (O), et b étant le coefficient d'atténuation de l'étalon,
   — la comparaison des signaux $Y_{ij}$ avec les signaux d'atténuation $b \times X_{ij}$ pour en déduire, pour chaque canal de rang i, des coefficients $A_{ik}$ du polynôme $P_n^i$ de degré n tel que :

$$P_n^i \ (Y_{ij}) = \sum_{k=o}^{k=n} A_{ik} \ Y_{ij}^k$$

de manière à obtenir une approximation polynomiale des valeurs calculées $X_{ij}$ suivant une loi d'écart déterminée.

2. Procédé selon la revendication 1, caractérisé en ce que la distance $X_{ij}$ est calculée par la formule :

$$\sqrt{R_2 - \{|FO| \sin \delta_i - r \cos (\Phi - ß_j - \delta_i)\}^2} \quad (1)$$

R étant le rayon de l'étalon (32), FO étant la distance entre la source de rayons X et l'axe de rotation (O) et $\delta_i$ étant l'angle entre le rayon passant par l'axe de rotation (O) et le rayon passant par le canal d'ordre i.

3. Procédé selon la revendication 1 ou 2, caractérisé en ce que le calcul des coefficients $A_{ik}$ du polynome $P_n^i$ (Y) est effectué pour que l'écart quadratique :

$$C_i = \sum_{j=1}^{m} \ \{ \sum_{k=o}^{n} \ (A_{ik} \ Y_{ij}^k) - b \cdot X_{ij}\}^2 \qquad (2)$$

soit aussi faible que possible.

4. Appareil d'étalonnage d'un scanner à rayons X qui comporte une source de rayons X (30) et un dispositif de détection (31) de i = 1 à N détecteurs ou canaux portés par une structure qui tourne autour d'un axe (0), en utilisant un étalon circulaire (32), caractérisé en ce qu'il comprend :
   — des moyens pour disposer au moins un étalon circulaire (32) entre la source de rayons X et le dispositif de détection dans une position excentrée par rapport à l'axe de rotation (0) de la structure tournante,
   — des moyens pour déplacer le scanner de j = 1 à m positions angulaires différentes $ß_j$,
   — des moyens (51, 52) pour calculer pour chaque position $ß_j$, à partir des signaux reçus sur les N détecteurs, N signaux $Y_{ij}$ représentatifs de l'atténuation subie par le rayonnement X dans l'étalon,

— des moyens (53) pour enregistrer les N signaux $Y_{ij}$ pour chacune des m positions angulaires $\beta_j$,

— des moyens (55) pour calculer pour toutes les positions angulaires j = 1 à m, à partir de signaux r et $\Phi$ de décentrage, des signaux $X_{ij}$ représentatifs de la longueur des trajets du rayonnement X dans l'étalon pour les canaux i = 1 à N ainsi que les signaux d'atténuation correspondants b · $X_{ij}$, r et $\Phi$ étant les coordonnées polaires du centre (E) de l'étalon par rapport à un système de coordonnées lié audit axe de rotation (0), b étant le coefficient d'atténuation de l'étalon,

— des moyens pour comparer les signaux $Y_{ij}$ avec les signaux d'atténuation b · $X_{ij}$ et pour calculer des signaux représentatifs de coefficients $A_{ik}$ du polynôme $P_n^i$ de degré n tel que :

$$P_n^i \ (Y_{ij}) \ = \ \sum_{k=o}^{k=n} \ A_{ik} \ Y_{ij}^k$$

de manière à obtenir une approximation polynomiale des valeurs calculées $X_{ij}$ suivant une loi d'écart déterminée,

— des moyens (59) pour enregistrer lesdits coefficients $A_{ik}$ ainsi calculés de manière à les utiliser ensuite comme coefficients multiplicateurs des atténuations $Y_{ij}$ subies par le rayonnement X dans le corps du patient.

## Claims

1. A method for the calibration of an x-ray scanner, which comprises a source (30) of x-rays and a detection device (31) with i = 1 to N detectors or channels arranged on a structure which revolves about an axis (O), using a circular standard (32), characterized in that it comprises the following operations :

— the putting into position of a circular standard (32) whose center (E) is arranged in a position eccentric in relation to the axis of rotation of the scanner,

— the displacement of the scanner through j = 1 to m different angular positions $\beta_j$,

— the calculation on the basis of the signals received from the detectors by a channel of the rank i and for each angular position $\beta_j$ of the scanner, of signals $Y_{ij}$ representative of the attenuation undergone by the x-ray radiation in the standard,

— the registering of the N · m signals $Y_{ij}$,

— the calculation, on the basis of the signals r and $\Phi$ of de-centering, of the signals $X_{ij}$ representative of the distance traveled by the x-ray radiation in the standard and also the corresponding attenuation signals b · $X_{ij}$, r and $\Phi$ being the polar coordinates of the center (E) of the standard in relation to a system of coordinates tied to the said axis (O) of rotation, and b being the coefficient of attenuation of the standard,

— the comparison of the signals $Y_{ij}$ with the attenuation signals b · $X_{ij}$ in order to deduce therefrom, for each channel of the rank i, the coefficients $A_{ik}$ of the polynomial $p_n^i$ of the degree n such that :

$$p_n^i \ (Y_{ij}) \ = \ \sum_{k=0}^{k=n} \ A_{ik} \ Y_{ij}^k$$

in such a manner as to produce a polynomial approximation of the calculated values $X_{ij}$ in accordance with a predetermined law of divergence.

2. The method as claimed in claim 1, characterized in that the distance $X_{ij}$ is calculated by the formula :

$$\sqrt{R^2 - \{|FO| \sin \delta_1 \ - r \cos (\Phi - \beta_j - \delta_i)\}^2} \quad (1)$$

R being the ray from the standard (32), FO being the distance between the x-ray source and the axis of rotation (0) and $\delta_i$ being the angle between the ray passing through the axis of rotation (0) and the ray passing through the channel of the order of i.

3. The method as claimed in claim 1 or claim 2, characterized in that the calculation of the coefficients $A_{ik}$ of the polynomial $p_n^i$ (Y) is so performed that the quadratic divergence :

$$c_i = \sum_{j=1}^{\blacksquare} \left( \sum_{k=0}^{n} (A_{ik}\, Y_{ij}^k) - b \cdot X_{ij} \right)^2 \qquad (2)$$

is a small as possible.

4. A calibration apparatus for an x-ray scanner, which comprises an x-ray source (30) and a detection device (31) of i = 1 to N detectors or channels arranged on a structure which revolves about an axis (0), using a circular standard (32), characterized in that it comprises :

— means to arrange at least one circular standard (32) between the x-ray source and the detection device in an eccentric position in relation to the axis (0) of rotation of the revolving structure,

— means for displacement of the scanner through j = 1 to m different angular positions $\beta_j$,

— means (51 and 52) in order to calculate for each position $\beta_j$, on the basis of the signals received at the N detectors, N signals $Y_{ij}$ representative of the attenuation undergone by the x-ray radiation in the standard,

— means (53) in order to register the N signals $Y_{ij}$ for each of the m angular positions $\beta_j$,

— means (53) in order to calculate, for all the angular positions j = 1 to m and starting from the r and $\Phi$ de-centering signals, signals $X_{ij}$ representative of the length of the x-ray paths in the standard for the channels i = 1 to N and the corresponding attenuation signals $b \cdot X_{ij}$, r and $\Phi$ being the polar coordinates of the center (E) of the standard in relation to a system of coordinates tied to the said axis (0) of rotation, b being the coefficient of attenuation of the standard,

— means for comparison of the signals $Y_{ij}$ with the signal of attenuation $b \cdot X_{ij}$ and in order to calculate signals representative of the coefficients $A_{ik}$ of the polynomial $P_n^i$ of the degree n such that

$$P_n^i (Y_{ij}) = \sum_{k=0}^{k=n} A_{ik}\, Y_{ij}^k$$

in such a manner as to produce a polynomial approximation of the calculated values $X_{ij}$ following a pre-determined law of divergence,

— and means (59) in order to register the said coefficients $A_{ij}$ calculated in this manner and to then use them as multiplying coefficients for the attenuations $Y_{ij}$ undergone by the x-rays in the body of the patient.

## Patentansprüche

1. Eichungsverfahren für einen Röntgenstrahl-Scanner mit einer Röntgenstrahlquelle (30) und einer Detektorvorrichtung (31) mit i = 1 bis N Detektoren oder Kanälen, die von einem Aufbau, der sich um eine Achse (0) dreht, gehalten wird, wobei ein kreisförmiger Eichkörper (32) verwendet wird, dadurch gekennzeichnet, daß es die folgenden Operationen umfaßt :

— Einsetzen eines kreisförmigen Eichkörpers (32), dessen Mittelpunkt (E) an einer in bezug auf die Drehachse des Skanners exzentrischen Position angeordnet wird,

— Verschieben des Skanners an j = 1 bis m verschiedene Winkelpositionen $\beta_j$,

— Berechnen von Signalen $Y_{ij}$, die die Dämpfung der Röntgenstrahlung im Eichkörper darstellen, anhand der von den Detektoren durch den Kanal des Rangs i empfangenen Signale und für jede Winkelposition $\beta_j$ des Skanners,

— Eintragen der N · m Signale $Y_{ij}$,

— Berechnen der Signale $X_{ij}$, die den von der Röntgenstrahlung im Eichkörper durchlaufenen Abstand darstellen, sowie von entsprechenden Dämpfungssignalen $b \cdot X_{ij}$ anhand von Dezentrierungssignalen r und $\Phi$, wobei r und $\Phi$ die Polarkoordinaten des Zentrums E des Eichkörpers in bezug auf ein auf die Drehung (O) bezogenes Koordinatensystem und b der Dämpfungskoeffizient des Eichkörpers sind,

— Vergleichen der Signale $Y_{ij}$ mit den Dämpfungssignalen $b \cdot X_{ij}$, um daraus für jeden Kanal vom Rang i die Koeffizienten $A_{ik}$ des Polynoms $P_n^i$ vom Grad n, mit :

$$P_n^i (Y_{ij}) = \sum_{k=0}^n A_{ik} \cdot Y_{ij}^k,$$

abzuleiten, so daß eine Polynomapproximation der berechneten Werte $X_{ij}$ gemäß einem gegebenen Abweichungsgesetz erhalten wird.

2. Verfahren gemäß Anspruch 1, dadurch gekennzeichnet, daß der Abstand $X_{ij}$ durch die Formel

EP 0 346 181 B1

$$\sqrt{R^2 - \{|FO| \cdot \sin \delta_i - r \cdot \cos (\Phi - \beta_j - \delta_i)\}^2} \quad (1)$$

berechnet wird, wobei R der Radius des Eichkörpers (32), FO der Abstand zwischen der Röntgenstrahlquelle und der Drehachse (0) und δi der Winkel zwischen dem durch die Drehachse (O) gehenden Radius und dem durch den Kanal der Ordnung i gehenden Radius ist.

3. Verfahren gemäß Anspruch 1 oder 2, dadurch gekennzeichnet, daß die Berechnung der Koeffizienten $A_{ik}$ des Polynoms $P^i_n$ (Y) so ausgeführt wird, daß die quadratische Abweichung

$$c_i = \Sigma^m_{j=1} \{\Sigma^n_{k=0} (A_{ik} \cdot Y^k_{ij}) - b \cdot X_{ij}\}^2 \quad (2)$$

so klein wie möglich wird.

4. Eichungsgerät eines Röntgenstrahl-Scanners mit einer Röntgenstrahlquelle (30) und einer Detektorvorrichtung (31) mit i = 1 bis N Detektoren oder Kanälen, die von einem Aufbau, der sich um eine Achse (O) dreht, gehalten wird, wobei ein kreisförmiger Eichkörper verwendet wird, dadurch gekennzeichnet, daß es umfaßt:
— Mittel zum Anordnen wenigstens eines kreisförmigen Eichkörpers (32) zwischen der Röntgenstrahlquelle und der Detektorvorrichtung an einer in bezug auf die Drehachse (O) des drehbaren Aufbaus exzentrischen Position,
— Mittel zum Verschieben des Scanners an j = 1 bis m verschiedene Winkelpositionen $\beta_j$,
— Mittel (51, 52) zum Berechnen von N Signalen $Y_{ij}$, die die Dämpfung der Röntgenstrahlung im Eichkörper darstellen, anhand von in den N Detektoren empfangen Signalen für jede Position $\beta_j$,
— Mittel (53) zum Eintragen der N Signale $Y_{ij}$ für jede der m Winkelpositionen $\beta_j$,
— Mittel (55) zum Berechnen von Signalen $X_{ij}$, die die Länge der Bahnen der Röntgenstrahlung im Eichkörper für die Kanäle i = 1 bis N darstellen, sowie von entsprechenden Dämpfungssignalen $b \cdot X_{ij}$ anhand von Dezentrierungssignalen r und $\Phi$ für sämtliche Winkelpositionen j = 1 bis m, wobei r und $\Phi$ die Polarkoordinaten des Zentrums (E) des Eichkörpers in bezug auf ein auf die Drehachse (O) bezogenes Koordinatensystem und b der Dämpfungskoeffizient des Eichkörpers sind,
— Mittel zum Vergleichen der Signale $Y_{ij}$ mit den Dämpfungssignalen $b \cdot X_{ij}$ und zum Berechnen der Signale, die die Koeffizienten $A_{ik}$ des Polynoms $P^i_n$ vom Grad n, mit

$$P^i_n (Y_{ij}) = \Sigma^n_{k=0} A_{ik} \cdot Y^k_{ij},$$

darstellen, so daß eine Polynomapproximation der berechneten Werte $X_{ij}$ gemäß einem gegebenen Abweichungsgesetz erhalten wird,
— Mittel (59) zum Eintragen der auf diese Weise berechneten Koeffizienten $A_{ik}$, derart, daß sie anschließend als Multiplikationskoeffizienten der Dämpfungen $Y_{ij}$ der Röntgenstrahlung im Körper eines Patienten verwendet werden.

9

# FIG_1

# FIG_2

Atténuation
(Echelle logarithmique)

N° du canal

# FIG_3

Atténuation
(Echelle logarithmique)

24

23

C'

C

SC

---

REF

| 50 | 51 | 52 | | 53 | 54 | 55 |
|---|---|---|---|---|---|---|
| CODAGE | LOGA-RITHME | SOUS-TRACTION | | $Y_{ij}$ | CALCUL r et $\Phi$ | CALCUL $X_{ij}$ |

C1    CN

MEMOIRE

62

60

61    MEMOIRE

# FIG_5

58

CALCUL $A_{ik}$

59

CALCUL
CORRECTIONS

MEMOIRE

# FIG_4